# EUROPEAN PATENT SPECIFICATION

(11) **EP 0 632 131 B1**
(45) Date of publication and mention of the grant of the patent: **19.01.2000**
(21) Application number: 93916200.4
(22) Date of filing: 21.07.1993
(51) Int. Cl.: C12Q 1/18, C12M 1/34

(54) **METHOD OF EXAMINING WITH ANTIBACTERIAL AND APPARATUS THEREFOR**
VERFAHREN ZUR UNTERSUCHUNG MIT EINER ANTIBAKTERIELLEN AGENS UND VORRICHTUNG DAFÜR
PROCEDE ET APPAREIL D'EXAMEN RECOURANT A UN AGENT ANTIBACTERIEN

(30) Priority: 22.07.1992 JP 19546892
(43) Date of publication of application: 04.01.1995
(73) Proprietor: DAIKIN INDUSTRIES, LIMITED, Osaka-shi Osaka 530 (JP)
(72) Inventor: KUSUNOKI, Shin-ichiro, Nerima-ku, Tokyo 178 (JP); ARAI, Jun-ichiro, Tasukuba-shi, Ibaragi 305 (JP)
(74) Representative: Prüfer, Lutz H., Dipl.-Phys.
(86) International application number: JP9301017
(87) International publication number: WO9402632

(56) References cited:
- EP-A- 0 509 791
- WO-A-90/03441
- DD-A- 295 509
- JP-A- 1 222 767
- JP-A- 3 198 767
- JP-A- 53 099 379
- JP-A- 56 140 898
- US-A- 4 311 794
- US-A- 4 321 322

## Description

### Technical Field

This present invention relates to an antibacterial drug inspection method and apparatus therefor, and more particularly, this present invention relates to a method and apparatus for inspecting whether or not bacteria which is a cause of infectious disease and the like has drug sensitivity to specific antibacterial drug.

### Background Art

When medical care for infectious disease which is caused by bacteria is performed in clinical iatrology, it is widely employed as a general medical care method that dosage of antibacterial drug (antibiotic resistance, bactericidal drug and the like) to which bacteria causing infectious disease has drug sensitivity. But, it must be recognized prior to dosing the antibacterial drug that bacteria causing infectious disease has drug sensitivity to the antibacterial drug. Because, it is known that following general usage of antibiotic resistance, for example, for medical care of infectious disease, resistance bacteria having resistance to such antibiotic resistance appears. And because, when the most recent antibiotic resistance is applied to bacteria which has no resistance to all antibiotic resistance, resistance bacteria appears which has resistance to the most recent antibiotic resistance.

Minimum inhibitory concentration (MIC) measuring method established by Nihon Kagaku Ryouhou Gakkai and a method using liquid medium are proposed as drug sensitivity inspection method for satisfying such demand.

The MIC measuring method is a method for inoculating bacteria by concentration of 10⁶ articles/ml, for example, onto a agar medium including antibacterial resisrance by predetermined concentration, which bacteria is incubated for a predetermined time in another medium, for incubating the bacteria inoculated onto the agar medium for a predetermined time (about 18-20 hours), and for judging drug sensitivity of the corresponding bacteria by visually judging whether or not colonies are generated. Consequently, it is accurately judged that whether or not bacteria has drug sensitivity and that a degree of drug sensitivity of the bacteria, by preparing plural agar medium, antibacterial drug concentration of each agar medium being different from one another.

The method using liquid medium is a method for performing MIC method using liquid medium instead of the agar medium. The method judges whether or not bacteria has drug sensitivity and degree of drug sensitivity of the bacteria based upon turbidity of the liquid medium, variation in pH and the like.

Because any one of both above-mentioned methods is MIC method, essentially, an adjustment for separation bacteria share as a pretreatment requires extremely long time, and after the adjustment, the above-mentioned predetermined time is required. Thereby, a disadvantage arises in that a required time in its entirety becomes extremely long. Specifically, the required time is 18-20 hours after adjustment for separation bacteria share when the MIC method is employed, and the required time is several to 20 hours after adjustment for separation bacteria share when the method using liquid medium is employed. Especially, it is strongly demanded to shorten the above-mentioned required time, by taking into consideration that though judgement of drug sensitivity is necessary for determining drug which is to be dosed to infectious disease patient and the like, it is desired that drug sensitivity is judged within as short time as possible in view of early treatment.

Though the MIC measuring method visually judges generation of colonies, it may be thought that it is possible to automating the MIC measuring method at its entirety using a CCD camera and the like, for example. But, bacteria objected for drug sensitivity inspection for treamtment of infectious disease and the like, is not known its type, and manner of multiplication of bacteria greatly differs depending upon type of bacteria. Therefore, it is almost impossible that sensitivity adjustment and the like of a CCD camera is performed for adapting to a type of bacteria which is not known its type. Consequently, an apparatus is not supplied at all which automatically performs drug sensitivity inspection of antibacterial drug to bacteria of which type is not known. Therefore, an operation for performing drug sensitivity inspection is extremely complicated.

WO 90/03441 A1 discloses an inspection method for an antibacterial drug wherein oxygen in culture cells is determined.

JP 1-222767 A discloses a biosensor forming an electrode which can detect oxygen.

US 4,321,322 discloses the use of two electrodes for the same substance for measuring the current through the substance.

DD 295 509 A7 discloses a biosensor for quantitative determining of antibiotics wherein an electrode for detecting dissolved oxygen is used.

US 4,311,794 discloses the pressure measurement of oxygen produced during a catalase reaction the decomposition of peroxide. The pressure is monitored. The pressure is an indication for bacterial growth activity and absence of antibiotics.

EP 0 509 791 A1 (which is prior art under Art. 54(3) EPC for the contracting stated DE, FR, GB, IT) discloses a fluorescence measurement depending on the oxygen present for detection and evaluation of metabolic activity of microorganisms.

This present invention was made to solve the above-mentioned problems.

It is an object of the present invention to supply a novel antibacterial drug inspection method and apparatus therefor, the method and apparatus inspecting drug sensitivity of bacteria in a liquid objected for measurement to antibacterial drug with a short time, and automating a series of inspection.

To perform the above-mentioned object, an inspection method for an antibacterial drug according to claim 1 is provided.

Where, the antibacterial drug is used as an intension including antibiotic resistance, bactericidal drug and the like. As to adding order for existence the antibacterial drug and the bacteria in the liquid, one of the antibacterial drug and bacteria may be added prior to another, or both may be added simultaneously. Further, concentration of antibacterial drug to be added is preferably determined to sufficiently higher concentration (for example, about 10-100 times to usual concentration) than the usual concentration.

According to claims 1 or 2, rotenone, amytal, antimycin A, cyanogen compound and the like are exemplified as the respiration interfering drug, and oligomycin, atractyloside and the like are exemplified as the bacteria non-sensitive respiration interfering drug, body fluid, urine, lixiviation fluid, tissue fluid, tissue wash liquid, culture solution and the like are exemplified as the liquid in which bacteria exists.

An inspection apparatus for an antibacterial drug according to claim 3 is also provided to solve the object. Where, the term "antibacterial drug" is used as an intension including antibiotic resistance, bactericidal drug and the like. As to adding order for existence the antibacterial drug and the bacteria in the liquid, one of the antibacterial drug and bacteria may be added prior to another, or both may be added simultaneously. Further, concentration of antibacterial drug to be added is preferably determined to sufficiently higher concentration (for example, about 10-100 times to usual concentration) than the usual concentration. According to claims 3 or 4 rotenone, amytal, antimycin A, cyanogen compound and the like are exemplified as the respiration interfering drug, and oligomycin, atorakuchirosido and the like are exemplified as the bacteria non-sensitive respiration interfering drug.

As to the antibacterial drug inspection method according to claim 1, when the bacteria in the liquid has drug sensitivity to the corresponding antibacterial drug, consumption quantity of the dissolved oxygen in the liquid decreases caused by decrease of a living number of bacteria. On the contrary, when the bacteria in the liquid has no drug sensitivity to the corresponding antibacterial drug, and when no antibacterial drug exist in the liquid, consumption quantity of the dissolved oxygen in the liquid increases following passage of the time caused by multiplication of bacteria. Therefore, it is judged whether or not the bacteria in the liquid has drug sensitivity to the corresponding antibacterial drug by judging whether or not the dissolved oxygen quantities are essentially different in case that the antibacterial drug exists in the liquid and in case that no antibacterial drug exist in the liquid. Further, as is apparent from the above description, it is sufficient that dissolved oxygen quantity is measured which varies following respiration of bacteria, a required time after adjustment for separation bacteria share is extremely shortened.

As to the antibacterial drug inspection method according to claim 1 or 2, because the respiration interfering drug and/or the bacteria non-sensitive respiration interfering drug is existed in the liquid in which bacteria exists, influence of respiration of animal cell other than the bacteria is eliminated, and judgement accuracy of drug sensitivity is improved. When the respiration interfering drug is existed in the liquid, it may be thought that respiration of both bacteria and animal cell other than bacteria is interfered. But, as a result of researches the inventors have earnestly performed, when the respiration interfering drug by a usual concentration (for example, about 10-100 µM) is used, sufficient respiration interfering function to the animal cell is recognized, while scarce respiration interfering function to the bacteria is recognized. Therefore, when the respiration interfering drug is used in addition to or instead of the bacteria non-sensitive respiration interfering drug, influence of respiration of only animal cell is securely eliminated, and judging accuracy of drug sensitivity is improved.

As to the antibacterial drug inspection apparatus according to claim 3, the antibacterial drug is existed in culture cell means exept one of at least one culture cell means, in this condition liquid in which bacteria exists is supplied to each culture cell means by the test solution injection means. Dissolved oxygen quantities in the culture cell means are measured by the oxygen electrode means, each oxygen electrode means corresponding to each culture cell means. And, it is judged by the judgement means whether or not the output signal from the oxygen electrode means corresponding to the one culture cell means and the output from the oxygen lectrode means corresponding to other culture cell means are essentially different from one another. When both output signal are not essentially different, it is judged that the bacteria has no drug sensitivity to the antibacterial drug, on the contrary, when both output signal are essentially different, it is judged that the bacteria has drug sensitivity to the antibacterial drug. Further, as is apparent from the above description, it is sufficient that dissolved oxygen quantity is measured which varies following respiration of bacteria, a required time after adjustment for separation bacteria share is extremely shortened. Furthermore, it is sufficient that the dissolved oxygen quantities corresponding to exisence and non-existence of the antibacterial drug are measured, and it is judged whether or not both measurement signals are different from one another, it is easily and accurately judged the drug sensitivity of the bacteria objected for inspection even when type of the bacteria is not known, Therefore, the antibacterial drug inspection apparatus can easily be automated at its entirety.

As to the antibacterial inspection apparatus according to claim 3 or 4, because respiration interfering drug and/or bacteria non-sensitive respiration interfering drug are existed in all culture cell means, influence of respiration of animal cell other than the bacteria is eliminated, and judgement accuracy of drug sensitivity is improved.

### Brief Description of The Drawings

Figure 1 is a flowchart for explaining inspection sequence of an embodiment of an antibacterial drug inspention method according to the present invention;
Figure 2 is a schematic view illustrating an embodiment of an antibacterial drug inspection apparatus of the present invention;
Figure 3 is a flowchart for explaining inspection sequence of another embodiment of an antibacterial drug inspention method according to the present invention;
Figure 4 is a schematic view illustrating another embodiment of an antibacterial drug inspection apparatus of the present invention;
Figure 5 is a diagram illustrating variation of dissolved oxygen concentration following passage of the time in case that E. coli C#2021 is used as bacteria;
Figure 6 is a diagram illustrating variation of dissolved oxygen concentration following passage of the time in case that staphylococcus aureus is used as bacteria;
Figure 7 is a diagram illustrating variation of dissolved oxygen concentration following passage of the time in case that E. coli C#1181 is used as bacteria.

### Best mode for Utilizing The Invention

Referring to the attached drawings, we explain the present invention in detail.

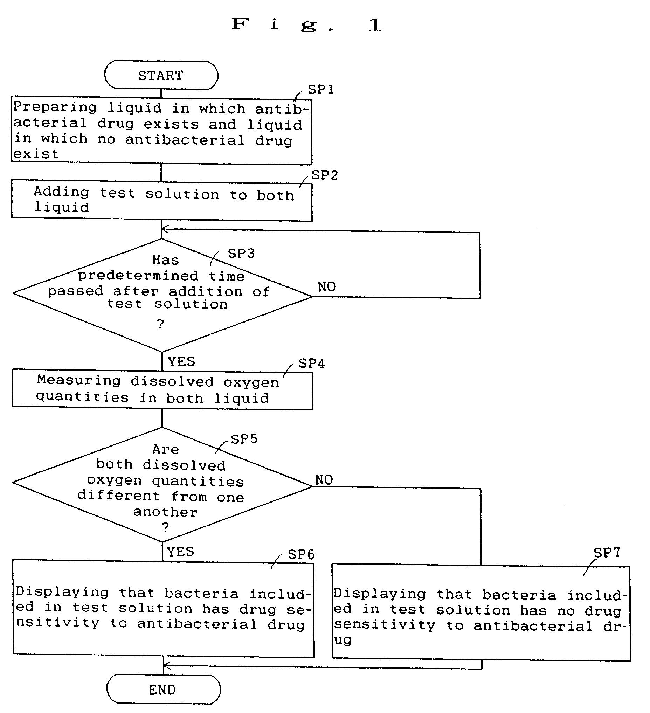

Figure 1 is a flowchart for explaining inspection sequence of an embodiment of an antibacterial drug inspention method according to the present invention.

In step SP1, a liquid in which antibacterial drug by predetermined concentration (extremely higher concentration than usual concentration) exists and a liquid in which no antibacterial drug exist therein are prepared, in step SP2, a test solution is added to both liquid, in step SP3, passage of predetermined time after addition of the test solution is waited, in step SP4, dissolved oxygen quantities in both liquid are measured, in step SP5, it is judged whether or not both dissolved oxygen quantities are essentially different from one another, when both dissolved oxygen quantities are essentially different from one another, in step SP6, it is displayed that bacteria included in the test solution has drug sensitivity to the antibacterial drug. On the contrary, when it is judged in step SP5 that both dissolved oxygen quantities are essentially equal to one another, in step SP7, it is displayed that bacteria included in the test solution has no drug sensitivity to the antibacterial drug.

In above-mentioned step SP5, it is judged whether or not both dissolved oxygen quantities are essentially different from one another, for the sake of eliminating influence of error of measurement system and the like.

In this embodiment, judgement whether or not bacteria has drug sensitivity is performed by waiting till the dissolved oxygen quantities can be judged to be essentially different from one another, drug sensitivity inspection result can be obtained within 60 minutes at the longest after addition of the test solution.

Further, in this embodiment, drug sensitivity is inspected for one species of antibacterial drug. When drug sensitivity is inspected for more than two species of antibacterial drug, it is sufficient that plural liquids are prepared, each antibacterial drug existing in each liquid, and that dissolved oxygen quantities of the liquid in which antibacterial drug exists and of the liquid in which no antibacterial drug exists are determined, and that it is judged whether or not the former dissolved oxygen quantity and the latter dissolved oxygen quantity are essentially different from one another. For example, as the judgement sequence, comparing the dissolved oxygen quantities may sequentially be performed for every liquid in which no antibacterial drug exist, or comparing the dissolved oxygen quantities may simultaneously be performed for all liquids in which no antibacterial drug exist. In both judging sequences, drug sensitivity to more than two species of antibacterial drug can be inspected almost simultaneously. Of course, it is possible that the liquid in which antibacterial drug exists and the liquid in which no antibacterial drug exist are prepared as pairs.

### Second Embodiment

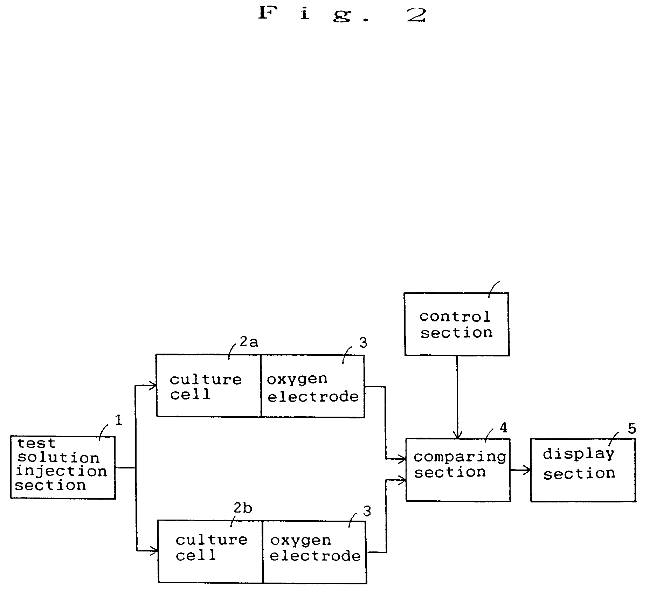

Figure 2 is a schematic view illustrating an embodiment of an antibacterial drug inspection apparatus of the present invention.

The antibacterial drug inspection apparatus includes two culture cells 2a and 2b, a test solution injection section 1 for adding test solution to the culture cells 2a and 2b simultaneously, oxygen electrodes 3, each oxygen electrode being provided corresponding to each of both culture cells 2a and 2b, a comparing section 4 for comparing output signals from both oxygen electrodes 3, and a display section 5 for displaying existence or not of drug sensitivity in response to comparison result of the comparing section 4. Antibacterial drug is existed in only one culture cell 2a. The comparing section 4 judges that both output signals sre different from one another only when a difference between both output signals is greater than a predetermined threashold value. Further, the antibacterial drug inspection apparatus includes a control section 6 for operating the comparing section 4 at a timing passed a predetermined time has passed after addition of the test solution.

As to the antibacterial drug inspection apparatus having the above-mentioned arrangement, the culture cell 2a in which antibacterial drug previously exists and the culture cell 2b in which no antibacterial drug exist are prepared, and in this condition, the test solution including bacteria is added to both culture cells 2a and 2b from the test solution injection section 1 simultaneously. Existence or non-existence of drug sensitivity of the bacteria is inspected as follows.

When the test solution is added, the antibacterial drug existing in the culture cell 2a begins acting to the bacteria in the test solution. When the bacteria has drug sensitivity, a living number of the bacteria decreases following passage of the time by the acting of the antibacterial drug. On the contrary, when the bacteria has no drug sensitivity and when the test solution is added to the culture cell in which no antibacterial drug exist, the living number of the bacteria increases following passage of the time. Thereby, consumption quantity of dissolved oxygen becomes small when the living number of bacteria decreases, while consumption quantity of dissolved oxygen becomes great when the living number of bacteria increases. Consequently, it is judged that whether or not both output signals are essentially different from one another, that is whether or not drug sensitivity exist, by measuring dissolved oxygen quantities in each culture cell with the oxygen electrodes 3 at the timing the predetermined time has passed after addition of the test solution, and by supplying the output signals from the oxygen electrodes 3 to the comparing section 4. Thereafter, existence or non-existence of drug sensitivity is displayed by the display section 5 in correspondence to the comparison result of the comparing section 4.

Further, in this embodiment, drug sensitivity is inspected for one species of antibacterial drug. When drug sensitivity is inspected for more than two species of antibacterial drug, it is sufficient that plural liquid are prepared, each antibacterial drug existing in each liquid, and that dissolved oxygen quantities of the liquid in which antibacterial drug exists and of the liquid in which no antibacterial drug exists, and that it is judged whether or not the former dissolved oxygen quantity and the latter dissolved oxygen quantity are essentially different from one another. For example, as the judgement sequence, comparing the dissolved oxygen quantities may sequentially be performed for every liquid in which no antibacterial drug exist, or comparing the dissolved oxygen quantities may simultaneously be performed for all liquid in which no antibacterial drug exist. In both judging sequences, drug sensitivity to more than two species of antibacterial drug can be inspected almost simultaneously. Of course, it is possible that the liquid in which the antibacterial drug exists and the liquid in which no antibacterial drug exist are prepared as pairs.

### Third Embodiment

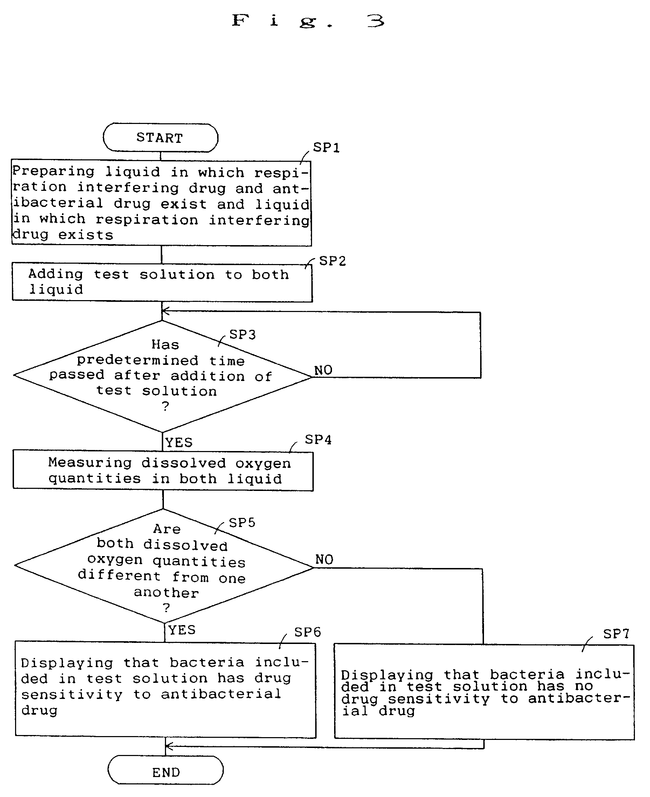

Figure 3 is a flowchart for explaining the inspection sequence of another embodiment of an antibacterial drug inspention method according to the present invention.

The flowchart differs from the flowchart in Fig. 1 only in that a respiration interfering drug or a bacteria non-sensitive respiration interfering drug is added to both liquid which are prepared in step SP1.

Therefore, in this embodiment, only the consumption quantity of dissolved oxygen (specifically, dissolved oxygen quantity at corresponding timing) caused by the living number of bacteria is measured by interfering respiration of animal cell other than bacteria in the test solution. Consequently, influence of the consumption of oxygen by animal cells is eliminated, and existence or non-existence of drug sensitivity is judged with high accuracy. Fourth Embodiment

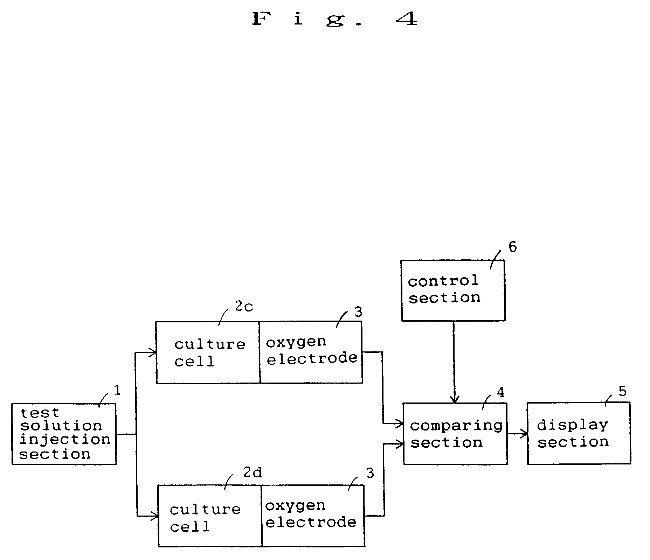

Figure 4 is a schematic view illustrating another embodiment of an antibacterial drug inspection apparatus of the present invention.

The apparatus differs from the embodiment in Fig. 2 only in that culture cells 2c and 2d in which a respiration interfering drug or a bacteria non-sensitive respiration interfering drug is added are used instead of the culture cells 2a and 2b.

Therefore, in this embodiment, only the consumption quantity of dissolved oxygen (specifically, dissolved oxygen quantity at corresponding timing) caused by the living number of bacteria is measured by interfering respiration of animal cell other than bacteria in the test solution. Consequently, influence of consumption of oxygen by animal cells is eliminated, and existence or non-existence of drug sensitivity is judged with high accuracy.

### Specific Example

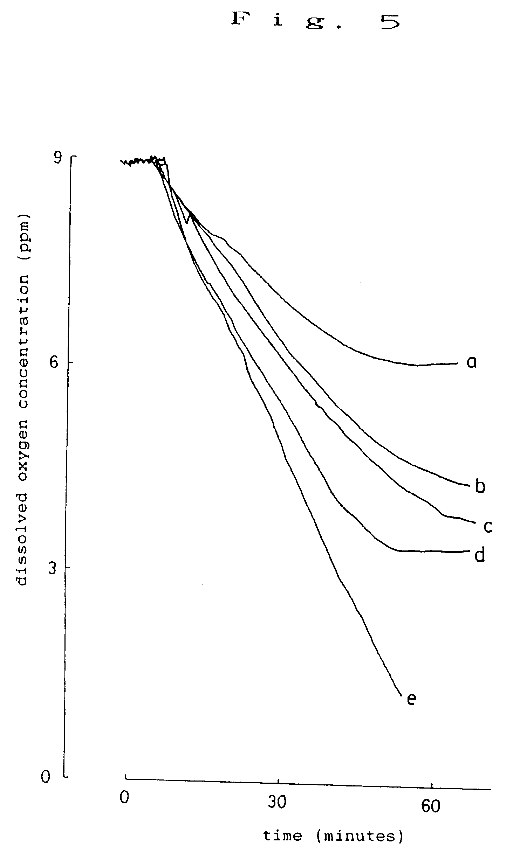

Figure 5 is a diagram illustrating variation of dissolved oxygen concentration in case that E. coli C#2021 (5x10⁶ articles/ ml) is used as bacteria, and that ampicillin (added 500 g/ml), streptomycin (added 200µg/ml), chloramphenicol (added 200µg/ml) and tetracycline (added 40µg/ml) are used as antibiotic resistance. In Fig. 5, a indicates ampicillin addition, b indicates streptomycin addition, c indicates chloramphenicol addition, d indicates tetracycline addition, and e indicates no antibiotic resistance addition.

As is apparent from Fig. 5, it is understood that the dissolved oxygen concentration almost linearly decreases following passage of the time when no antibiotic resistance is added, and that the dissolved oxygen concentration almost linearly decreases within some extent of time, thereafter decrease of the dissolved oxygen concentration is scarcely recognized, when antibiotic resistance is added. Further, linearly decreasing rates of a-d are smaller than the decreasing rate of e. Therefore, a-d and e are classified when 20-40 minutes has passed after addition of the test solution. That is, existence or non-existence of drug sensitivity is judged accurately with a short time.

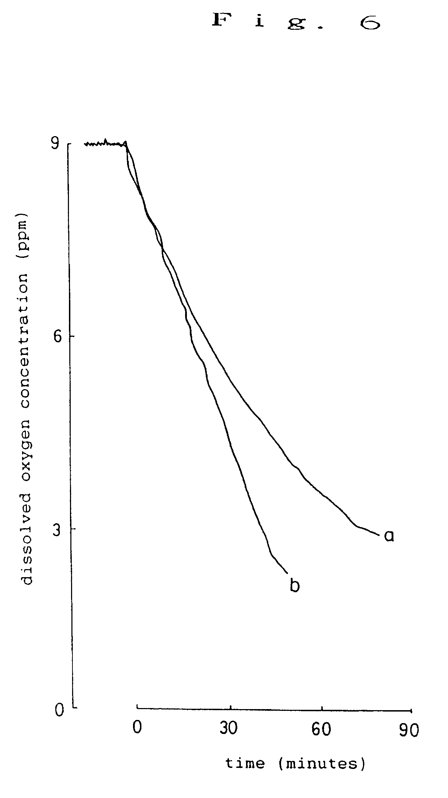

Figure 6 is a diagram illustrating variation of dissolved oxygen concentration in case that staphylococcus aureus (S. aureus) (1x10⁷ articles/ ml) is used as bacteria, and that ampicillin (added 1mg/ml) is used as antibiotic resistance. In Fig. 6, a indicates ampicillin addition, b indicates no antibiotic resistance addition.

As is apparent from Fig. 6, both can securely be classified when about 40 minutes has passed. Further, a dissolved oxygen concentration variation characteristic as similar as that of Fig. 6 is obtained when K. pneumoniae, P. aeruginosa are used.

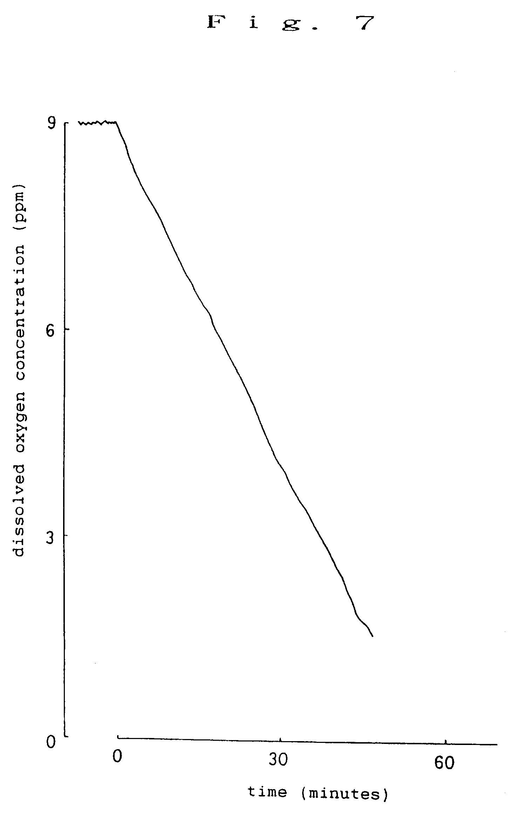

Figure 7 is a diagram illustrating variation of dissolved oxygen concentration in case that E. coli C#1181 (5x10⁶ articles/ ml) is used as bacteria, and that streptomycin (added 200µg/ml) is used as antibiotic resistance.

As is apparent from Fig. 7, E. coli C#1181 indicates reaction as similar as e in Fig. 5, it is understood that bacteria is not influenced in spite of addition of streptomycin. Thereby, non-existence of drug sensitivity is judged in this case.

Further, in the foregoing, description was made in case that antibiotic resistance is used as antibacterial drug, the inventors recognized that respiration of E. coli (1x10⁷ articles/ml) is interfered by using sulfanilamide (1mg/ml). Therefore, bacteria is died by interfering respiration. Also, imidazole derivative drugs other than sulfanilamide can be used as antibacterial drug, because imidazole derivative drug and the like stops consumption of oxygen by bacteria.

The present invention is not limited to the embodiments mentioned above. The present invention may add antibacterial drug after addition of the test solution. The present invention may also add the test solution and the antibacterial drug simultaneously. Further, various changes and modifications may be preferably made without departing from the spirit and scope of the invention.

### Possibility of Industrial Utilization

The present invention accurately judges with a short time whether or not working of micro-organism in living body system, reaction system and the like is suppressed by drug. Therefore, the present invention is applicable to preprocessing (inspection) of medical care.

## Claims

1. An inspection method for an antibacterial drug, comprising the steps of:
introducing an antibacterial drug into a first liquid in which bacteria exist,
detecting dissolved oxygen quantity on the first liquid and in a second liquid, in which bacteria but no antibacterial drug exist, determining whether or not the dissolved oxygen quantity varies in the first liquid, with respect to the dissolved oxygen quantity in the second liquid,
deciding that bacteria are drug sensitive to the corresponding antibacterial drug only when dissolved oxygen quantity in the first liquid varies with respect to the dissolved oxygen quantity in the second liquid, and
introducing a respiration interfering drug into the first and second liquids in which bacteria exist.

2. An antibacterial drug inspection method as set forth in claim 1, further comprising the step of:
introducing a bacteria non-sensitive respiration interfering drug into the first and second liquids in which bacteria exist.

3. An inspection apparatus for an antibacterial drug, comprising:
at least two culture cell means (2a, 2b, 2c, 2d) including a first and a second culture cell means (2a, 2b),
test solution injection means (1) for supplying a liquid in which bacteria exist to each culture cell means,
characterized by:
a plurality of oxygen electrode means (3) each corresponding to each culture cell means, and
comparing means (4) for comparing whether or not an output signal from the oxygen electrode means (3) for the liquid of the first culture cell means (2a) is different from an output signal from the oxygen electrode means (3) for the liquid of the second culture cell means (2b),
wherein antibacterial drug is absent in only the second culture cell means (2b), and
wherein all culture cell means (2c, 2d) comprise a respiration interfering drug therein.

4. The apparatus as set forth in claim 3, wherein all culture cell means (2c, 2d) comprise a bacteria non-sensitive respiration interfering drug therein.

## Patentansprüche

1. Untersuchungsverfahren für ein antibakterielles Arzneimittel, mit den Schritten:
Einbringen eines antibakteriellen Arzneimittels in eine erste Flüssigkeit, in der Bakterien vorhanden sind,
Ermitteln der Menge des gelösten Sauerstoffes in der ersten Flüssigkeit und in einer zweiten Flüssigkeit, in der Bakterien, aber kein antibakterielles Arzeimittel vorhanden sind,
Bestimmen, ob die Menge des gelösten Sauerstoffs in der ersten Flüssigkeit in Bezug auf die Menge des gelösten Sauerstoffs in der zweiten Flüssigkeit unterschiedlich ist,
Entscheiden, daß die Bakterien arzneimittelempfindlich gegen das entsprechende antibakterielle Arzeimittel sind, nur wenn die Menge des gelösten Sauerstoffs in der ersten Flüssigkeit in Bezug auf die Menge des gelösten Sauerstoffs in der zweiten Flüssigkeit unterschiedlich ist und
Einbringen eines die Atmung beeinflussenden Arzneimittels in die erste und zweite Flüssigkeit, in denen Bakterien vorhanden sind.

2. Untersuchungsverfahren eines antibakterielles Arzeimittels nach Anspruch 1, weiter mit dem Schritt:
Einbringen eines bakterienunempfindlichen, die Atmung beeinflussenden Arzneimittels in die erste und zweite Flüssigkeit, in denen Bakterien vorhanden sind.

3. Untersuchungsvorrichtung für ein antibakterielles Arzeimittel, mit:
mindestens zwei Kulturzellenmittel (2a, 2b, 2c 2d), welches ein erstes und ein zweites Kulturzellenmittel (2a, 2b) aufweist,
einem Testlösungsinjektionsmittel (1) zum Liefern einer Flüssigkeit, in der Bakterien vorhanden sind, an jedes Kulturzellenmittel,
gekennzeichnet durch:
einer Mehrzahl von Sauerstoffelektrodenmitteln (3), wobei jedes einem jeweiligen Kulturzellenmittel entspricht, und
einem Vergleichsmittel (4) zum Vergleichen, ob ein Ausgabesignal von-dem Sauerstroffelektrodenmittel (3) für die Flüssigkeit des ersten Kulturzellenmittels (2a) von einem Ausgabesignal von dem Sauerstoffelektrodenmittel (3) für die Flüssigkeit des zweiten Kulturzellenmittels (2b) verschieden ist oder nicht,
wobei das antibakterielles Arzeimittel nur in dem zweiten Kulturzellenmittel (2b) nicht vorhanden ist, und
wobei alle Kulturzellenmittel (2c, 2d) darin ein atmungsbeeinflussendes Arzneimittel enthalten.

4. Vorrichtung nach Anspruch 3, bei der alle Kulturzellenmittel (2c, 2d) ein bakterienunempfindliches, die Atmung beeinflussendes Arzneimittel darin enthalten.

## Revendications

1. Procédé de contrôle de l'action d'un médicament anti-bactérien comprenant les étapes :
d'introduction d'un médicament anti-bactérien dans un premier liquide dans lequel des bactéries ont été introduites,
de détection de la quantité de l'oxygène dissous dans le premier liquide et dans un second liquide, dans lequel les bactéries sont présentes, mais non le médicament anti-bactérien, de détermination si la quantité de l'oxygène dissous varie dans le premier liquide, par rapport à la quantité de l'oxygène dissous dans le second liquide, et
de décision que les bactéries sont sensibles au médicament anti-bactérien correspondant seulement lorsque la quantité de l'oxygène dissous dans le premier liquide varie par rapport à la quantité de l'oxygène dissous dans le second liquide, et
d'introduction d'un médicament agissant pour bloquer la respiration dans les premier et second liquides dans lesquels les bactéries ont été introduites.

2. Procédé du contrôle de l'action d'un médicament anti-bactérien selon la revendication 1, comprenant en outre l'étape :
d'introduction d'un médicament agissant pour bloquer la respiration non sensitive des bactéries dans les premier et second liquides dans lesquels les bactéries ont été introduites.

3. Appareil de contrôle de l'action d'un médicament anti-bactérien comprenant :
au moins deux moyens de cellules cultivées (2a, 2b, 2c, 2d) incluant un premier et un second moyens de cellules cultivées (2a, 2b),
un moyen d'injection de solution-test (1) pour introduction dans un liquide dans lequel des bactéries ont été introduites dans chaque moyen de cellules cultivées,
caractérisé par :
une pluralité de moyens d'électrode de détection de la teneur en oxygène (3), chacun correspondant à chaque moyen de cellules cultivées, et
un moyen de comparaison (4) pour comparer si un signal de sortie issu du moyen d'électrode (3) pour le liquide du premier moyen de cellule cultivée (2a) est ou non différent d'un signal de sortie issu du moyen d'électrode de détection de la teneur en oxygène (3) pour le liquide du second moyen de cellule cultivée (2b),
dans lequel le médicament anti-bactérien est absent seulement du second moyen de cellule cultivée (2b), et
dans lequel tous les moyens de cellules cultivées (2c, 2d) comprennent un médicament agissant pour y bloquer la respiration.

4. Appareil selon la revendication 3, dans lequel tous les moyens de cellules cultivées (2c, 2d) comprennent un médicament agissant pour y bloquer la respiration non sensitive des bactéries.
